# EUROPEAN PATENT APPLICATION

(11) **EP 4 248 867 A1**
(43) Date of publication of application: **27.09.2023**
(21) Application number: 22760098.8
(22) Date of filing: 24.02.2022
(51) Int. Cl.: A61B 5/327, A61B 5/346, A61B 5/332, A61B 5/00

(54) **SYSTEM AND METHOD FOR GENERATING ELECTROCARDIOGRAM ON BASIS OF DEEP LEARNING ALGORITHM**

(30) Priority: 24.02.2021 KR 20210024707
(71) Applicant: Medicalai Co., Ltd., Seoul 06302 (KR); Bodyfriend Co., Ltd., Seoul 06302 (KR)
(72) Inventor: KWON, Joon Myoung, Seoul 06629 (KR)
(74) Representative: Isarpatent
(86) International application number: PCT/KR2022/002747
(87) International publication number: WO 2022/182182

(57) **Abstract**

The present invention relates to a system and method for generating electrocardiograms based on a deep learning algorithm. The system includes: a data input unit configured to receive 12-lead electrocardiograms measured by a plurality of patients; a data extraction unit configured to extract training data from the input 12-lead electrocardiograms; a training unit configured to train a plurality of training models on a characteristic of the electrocardiograms by inputting the extracted training data to the plurality of training models; an electrocardiogram generation unit configured to receive at least one reference electrocardiogram from a subject to be measured, and to generate one or more virtual electrocardiograms by inputting the input reference electrocardiogram into the plurality of trained training models; and a control unit configured to synchronize the reference electrocardiogram and the generated virtual electrocardiograms with each other, and to output waveforms for the synchronized reference electrocardiogram and virtual electrocardiograms.

## Description

### Technical Field

The present invention relates to a system and method for generating electrocardiograms based on a deep learning algorithm, and more particularly, to a system and method for generating electrocardiograms that generate a plurality of electrocardiograms from one or more lead electrocardiograms by using a deep learning algorithm.

### Background Art

As for a standard 12-lead electrocardiogram used in hospitals, six electrodes are attached to the front of the chest, three electrodes (four electrodes when a ground electrode is included) are attached to the limbs, 12-lead information is all collected and combined, and then a disease is diagnosed.

A 12-lead electrocardiogram records potentials of the heart in 12 electrical directions centered on the heart. Through this, the disease of the heart confined to one area can be accurately diagnosed by determining the states of the heart in various directions.

The measurement of potentials of the heart in various directions is meaningful in that the characteristics of the heart can be determined in the individual directions. For this reason, medical professionals recommend the measurement of a standard 12-lead electrocardiogram for the diagnosis of heart disease (myocardial infarction, etc.).

However, it is difficult to measure a 12-lead electrocardiogram at home or in everyday life because the chest must be exposed to attach chest electrodes to take a 12-lead electrocardiogram and it is difficult for the general public to attach nine electrodes (three limb electrodes and six chest electrodes) to correct locations. In addition, it is difficult to use a 12-lead electrocardiogram for real-time monitoring because it is difficult to move after the attachment of ten electrodes.

Therefore, recently, a device capable of measuring a single-lead electrocardiogram or 2- or more-lead electrocardiograms has been developed such that it can be used in daily life.

First, a single-lead electrocardiogram device using two electrodes includes a watch-type electrocardiogram device (an Apple Watch or a Galaxy Watch). In the watch-type electrocardiogram device, the back of a watch and the left wrist come into contact with each other and the right finger and the crown of the watch come into contact with each other, so that a left arm electrode and a right arm electrode come into contact with each other and a lead I electrocardiogram is measured using the potential difference between the two electrodes.

In addition, a lead I electrocardiogram is measured by touching the crown with the right hand in a state in which the watch-type electrocardiogram device is worn on the left arm, a lead II electrocardiogram is measured by touching the crown with the right hand in a state in which the watch is placed on the stomach, and a lead III electrocardiogram is measured by touching the crown with the left hand in a state in which the watch is placed on the stomach. Then, a lead V1-6 electrocardiogram is measured by bringing the back of the watch into contact with V1-6 electrode positions in a state in which the left hand is in contact with the crown of the watch.

The above-described method has poor usability in that a user must accurately bring the electrocardiogram device into contact with the V1-6 electrode positions. The lead V1-6 electrocardiogram has a problem in which a standard chest lead electrocardiogram is not implemented in that it shows the potential difference between a right arm electrode and a V electrode, unlike the standard chest lead electrocardiogram that must show the potential difference between an imaginary central point and a V electrode even when a contact is made to a corresponding portion.

In addition, 2- or more-lead electrocardiograms can be measured in the state of holding the electrodes in both hands, bringing the electrodes on the back of the device into contact with the legs or ankles, and bringing the electrodes into contact with the left arm, the right arm, and the left leg, respectively. However, the above-described method is a method that cannot perform monitoring for a long time (1 hour, 24 hours, 7 days, or the like), and has a disadvantage in that the device itself must have three electrodes.

A background technology of the present invention is disclosed in Korean Patent No. 10-2180135 (published on November 17, 2020).

### Disclosure

### Technical Problem

Therefore, according to the present invention, there are provided a system and method for generating electrocardiograms that generate a plurality of electrocardiograms from one or more lead electrocardiograms by using a deep learning algorithm.

### Technical Solution

According to an embodiment of the present invention for overcoming the above technical problem, there is provided a system for generating electrocardiograms based on a deep learning algorithm, the system including: a data input unit configured to receive 12-lead electrocardiograms measured by a plurality of patients and patient information corresponding to the 12-lead electrocardiograms; a data extraction unit configured to classify and store the input 12-lead electrocardiograms according to the patient information, and to extract training data from the input 12-lead electrocardiograms; a training unit configured to train one or more training models on a characteristic of the electrocardiograms by inputting the extracted training data to the training models; an electrocardiogram generation unit configured to receive at least one reference electrocardiogram from a subject to be measured, and to generate one or more virtual electrocardiograms by inputting the input reference electrocardiogram into the trained training models; and a control unit configured to synchronize the reference electrocardiogram and the generated virtual electrocardiograms with each other, and to output waveforms for the synchronized reference electrocardiogram and virtual electrocardiograms.

The patient information may include at least one of gender, age, whether there is a heart disease, and the potential vector of each of the measured electrocardiograms.

The training unit may train a first training model to determine a potential vector for each of the input electrocardiograms and generate (12-n) electrocardiograms by inputting n lead electrocardiograms, which are some of 6-lead limb electrocardiograms and 6-lead chest electrocardiograms, to the first training model.

The training unit may construct 12 second training models for 6-lead limb electrocardiograms and 6-lead chest electrocardiograms, and may train the second training models to, when inputting the generated (12-n) electrocardiograms, output from the constructed first training model, to the second training models, convert the input lead electrocardiograms into styles each having a corresponding potential vector and output them as n virtual electrocardiograms.

The first and second training models may each be constructed by mixing a generative adversarial network and an autoencoder method.

The electrocardiogram generation unit may extract a potential vector for the reference electrocardiogram and generate a plurality of remaining virtual electrocardiograms by inputting the input reference electrocardiogram to the first training model, and may generate a virtual electrocardiogram having the same lead type as the reference electrocardiogram by inputting the generated virtual electrocardiograms to the second training models that have been trained on the extracted potential vector.

The control unit may synchronize the reference electrocardiogram and the plurality of virtual electrocardiograms by matching them, and may output the plurality of synchronized electrocardiograms through a monitor.

Furthermore, according to an embodiment of the present invention, there is provided a method for generating electrocardiograms using a system for generating electrocardiograms, the method including: receiving 12-lead electrocardiograms measured by a plurality of patients and patient information corresponding to the 12-lead electrocardiograms; classifying and storing the input 12-lead electrocardiograms according to the patient information, and extracting training data from the input 12-lead electrocardiograms; training one or more training models on a characteristic of the electrocardiograms by inputting the extracted training data to the training models; receiving at least one reference electrocardiogram from a subject to be measured, and generating one or more virtual electrocardiograms by inputting the input reference electrocardiogram into the trained training models; and synchronizing the reference electrocardiogram and the generated virtual electrocardiograms with each other, and outputting waveforms for the synchronized reference electrocardiogram and virtual electrocardiograms.

### Advantageous Effects

As described above, according to the present invention, a plurality of synchronized electrocardiograms may be generated from two electrocardiograms, measured at different points, by using a deep learning algorithm, so that the disease of the heart can be accurately diagnosed as in a 12-lead electrocardiogram.

In addition, according to the present invention, measurement may be performed at home or in daily life because two lead electrocardiograms are used, real-time monitoring may be enabled because measurement can be performed even in a moving state, and medical staff may be enabled to read synchronized lead electrocardiogram information tailored to a corresponding beat because synchronized electrocardiograms are generated utilizing electrocardiogram information measured at different times, thereby enabling more accurate diagnosis based on this.

### Description of Drawings

FIG. 1 is a block diagram illustrating a system for generating electrocardiograms according to an embodiment of the present invention;
FIG. 2 is a flowchart illustrating a method for generating electrocardiograms using a system for generating electrocardiograms according to an embodiment of the present invention;
FIG. 3 is an exemplary diagram illustrating a method of measuring a general 12-lead electrocardiogram;
FIG. 4 is an exemplary diagram illustrating step S240 shown in FIG. 2; and
FIG. 5 is an exemplary view illustrating step S260 shown in FIG. 2.

### Best Mode

A system 100 for generating electrocardiograms according to an embodiment of the present invention includes a data input unit 110, a data extraction unit 120, a training unit 130, an electrocardiogram generation unit 140, and a control unit 150.

First, the data input unit 110 receives 12-lead electrocardiograms measured through a plurality of patients and patient information corresponding to the 12-lead electrocardiograms.

In this case, the 12-lead electrocardiograms each include a 6-lead limb electrocardiogram and a 6-lead chest electrocardiogram, and the patient information includes at least one of gender, age, whether there is a heart disease, and the potential vector of each of the measured electrocardiograms.

The data extraction unit 120 classifies the 12-lead electrocardiograms using the input patient information, and stores them in a database. Then, the data extraction unit 120 randomly extracts a plurality of 12-lead electrocardiograms stored in the database, and generates training data.

Thereafter, the training unit 130 trains constructed training models using the training data. In other words, the training unit 130 constructs a first training model for extracting potential vector information for the input lead electrocardiograms and generating (12-n) lead virtual electrocardiograms from n lead reference electrocardiograms, and second training models for generating n lead virtual electrocardiograms from the (12-n) lead virtual electrocardiograms.

In addition, the training unit 130 trains the constructed first training model or second training models by inputting the generated training data to the first training model or second training models.

The electrocardiogram generation unit 140 obtains at least one reference electrocardiogram obtained from a subject to be measured. Then, the electrocardiogram generation unit 140 generates a first virtual electrocardiogram based on a potential vector for the reference electrocardiogram by inputting the obtained reference electrocardiogram to the first training model that has been trained. The electrocardiogram generation unit 140 causes the second training models to output second virtual electrocardiograms by inputting the generated first virtual electrocardiogram to the trained second training models.

Finally, the control unit 150 synchronizes the reference electrocardiogram and the virtual electrocardiograms with each other, and outputs a plurality of synchronized electrocardiograms from the reference electrocardiogram and the virtual electrocardiograms through a monitor device.

### Mode for Invention

Hereinafter, preferred embodiments according to the present invention will be described in detail with reference to the accompanying drawings. In this process, the thicknesses of lines or the sizes of components shown in the drawings may be exaggerated for clarity and convenience of description.

Furthermore, the terms to be described later are terms defined by taking into consideration the functions thereof in the present invention, which may vary according to the intention or custom of a user or an operator. Therefore, the definitions of these terms should be made based on the content described throughout the present specification.

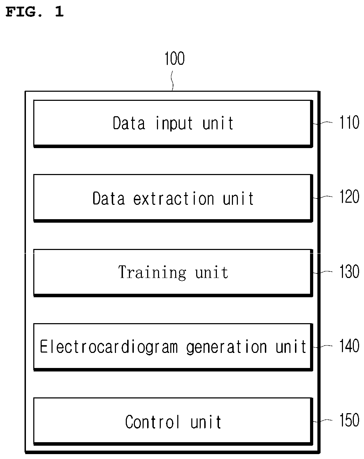

Hereinafter, a system for generating electrocardiograms based on a deep learning algorithm according to an embodiment of the present invention will be described in more detail with reference to FIG. 1.

FIG. 1 is a block diagram illustrating a system for generating electrocardiograms according to an embodiment of the present invention.

As shown in FIG. 1, a system 100 for generating electrocardiograms according to an embodiment of the present invention includes a data input unit 110, a data extraction unit 120, a training unit 130, an electrocardiogram generation unit 140, and a control unit 150.

First, the data input unit 110 receives 12-lead electrocardiograms measured through a plurality of patients and patient information corresponding to the 12-lead electrocardiograms.

In this case, the 12-lead electrocardiograms each include a 6-lead limb electrocardiogram and a 6-lead chest electrocardiogram, and the patient information includes at least one of gender, age, whether there is a heart disease, and the potential vector of each of the measured electrocardiograms.

The data extraction unit 120 classifies the 12-lead electrocardiograms using the input patient information, and stores them in a database. Then, the data extraction unit 120 randomly extracts a plurality of 12-lead electrocardiograms stored in the database, and generates training data.

Thereafter, the training unit 130 trains constructed training models using the training data. In other words, the training unit 130 constructs a first training model for extracting potential vector information for the input lead electrocardiograms and generating (12-n) lead virtual electrocardiograms from n lead reference electrocardiograms, and second training models for generating n lead virtual electrocardiograms from the (12-n) lead virtual electrocardiograms.

In addition, the training unit 130 trains the constructed first training model or second training models by inputting the generated training data to the first training model or second training models.

The electrocardiogram generation unit 140 obtains at least one reference electrocardiogram obtained from a subject to be measured. Then, the electrocardiogram generation unit 140 generates a first virtual electrocardiogram based on a potential vector for the reference electrocardiogram by inputting the obtained reference electrocardiogram to the first training model that has been trained. The electrocardiogram generation unit 140 causes the second training models to output second virtual electrocardiograms by inputting the generated first virtual electrocardiogram to the trained second training models.

Finally, the control unit 150 synchronizes the reference electrocardiogram and the virtual electrocardiograms with each other, and outputs a plurality of synchronized electrocardiograms from the reference electrocardiogram and the virtual electrocardiograms through a monitor device.

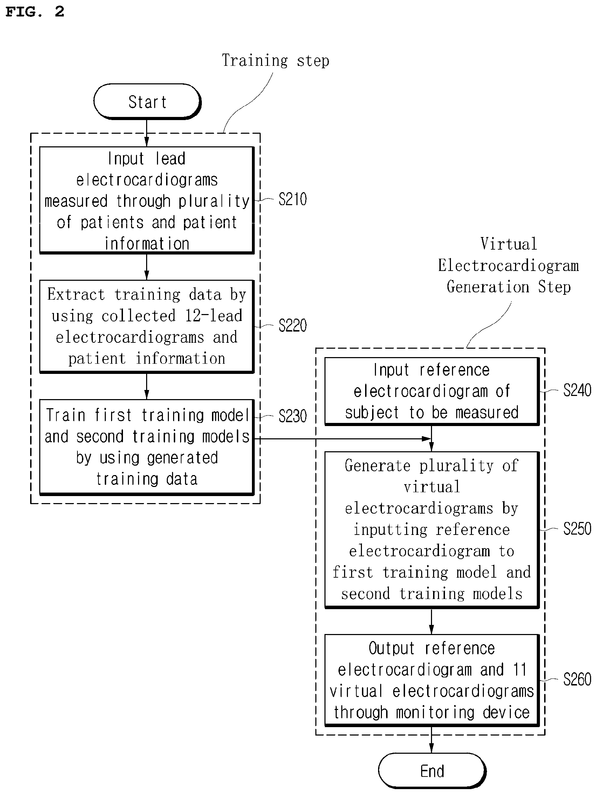

Hereinafter, a method for generating electrocardiograms using the system 100 for generating electrocardiograms according to an embodiment of the present invention will be described in more detail with reference to FIGS. 2 to 5.

FIG. 2 is a flowchart illustrating the method for generating electrocardiograms using the system for generating electrocardiograms according to the embodiment of the present invention.

As shown in FIG. 2, the method for generating electrocardiograms using the system for generating electrocardiograms according to the embodiment of the present invention is divided into the step of training training models and the step of generating electrocardiograms using the trained models.

In the step of training training models, first, the system 100 for generating electrocardiograms receives lead electrocardiograms measured through a plurality of patients and patient information in step S210.

First, a 12-lead electrocardiogram shows the potentials of the heart that are recorded in 12 electrical directions centered on the heart.

As for a general method of taking electrocardiograms, first, four electrodes (limb leads) are attached to both arms and legs of a patient. In this case, an electrocardiogram measurement device measures electric potentials with both arm and left leg electrodes, and a right leg electrode serves as a ground electrode.

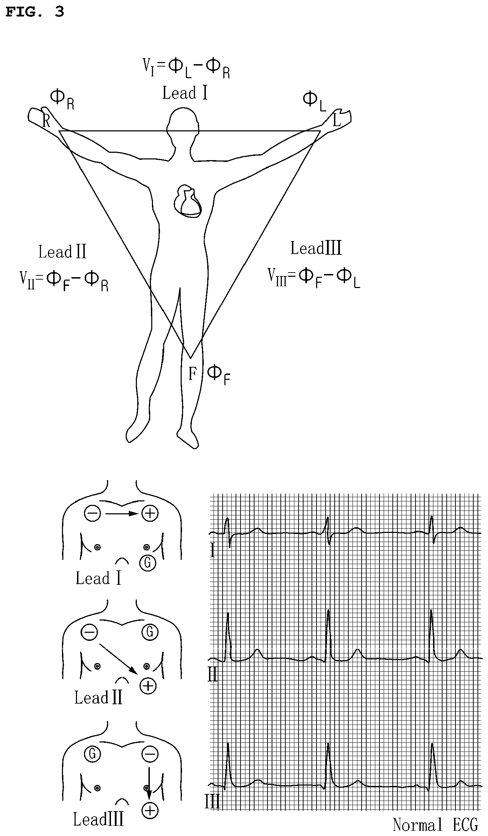

FIG. 3 is an exemplary diagram illustrating a method of measuring a general 12-lead electrocardiogram.

As shown in FIG. 3, the electrocardiogram measurement device generates a lead I electrocardiogram by subtracting the electric potentials of the right arm from the electric potentials of the left arm, generates a lead II electrocardiogram by subtracting the electric potentials of the right arm from the electric potentials of the left arm, and generates a lead III electrocardiogram by subtracting the electric potentials of the left arm from the electric potentials of the left leg.

The electrocardiogram measurement device obtains the electric potentials of an imaginary central point by calculating the averages of the electric potentials of both arm and left leg electrodes. In addition, the electrocardiogram measurement device generates a lead aVL electrocardiogram by subtracting the electric potentials of the virtual center point from the electric potentials of the left arm, and generates a lead aVR electrocardiogram by subtracting the electric potentials of the virtual center point from the electric potentials of the right arm. Furthermore, the electrocardiogram measurement device generates a lead aVF electrocardiogram by subtracting the electric potentials of the imaginary central point from the electric potentials of the left leg electrode. As described above, the electrocardiogram measurement device generates a total of six lead (six line) electrocardiograms using three limb electrodes (four electrodes when a ground electrode is included) .

Thereafter, the electrocardiogram measurement device generates a chest 6-lead electrocardiogram using potential differences between the electric potentials of the virtual central point determined at limb electrodes and the electric potentials of six electrodes attached to the chest. In other words, six electrodes V1, V2, V3, V4, V5, and V6 are attached from a location in front of a predetermined location to the left chest.

In addition, the electrocardiogram measurement device generates a lead V1 electrocardiogram by subtracting the electric potentials measured at an electrode V1 and the electric potentials of the virtual center point obtained through the averages of the limb electrodes above from each other.

As described above, the electrocardiogram measurement device generates 12-lead electrocardiograms using leads attached to a plurality of patients. Then, the generated 12-lead electrocardiograms are transferred to the system 100 for generating electrocardiograms.

In this case, the system 100 for generating electrocardiograms receives the generated 12-lead electrocardiograms and additional patient information corresponding thereto.

In this case, the patient information includes at least one of gender, age, whether there is a heart disease, and the potential vector of each of the measured electrocardiograms.

When step S210 is completed, the system 100 for generating electrocardiograms extracts training data using the collected 12-lead electrocardiograms and patient information in step S220.

In other words, the data extraction unit 120 classifies the collected 12-lead electrocardiograms according to the patient information and stores them in a database. Then, the data extraction unit 120 generates training data by randomly extracting a plurality of stored 12-lead electrocardiograms.

Thereafter, the training unit 130 trains a first training model and second training models using the generated training data in step S230.

First, the training unit 130 trains the first training model and the second training models on the characteristics of the electrocardiograms by inputting the training data composed of 12-lead electrocardiograms to the first training model and the second training models. In other words, in the lead electrocardiograms, an electric flow direction varies depending on the potential vector, and an electrocardiogram style is affected by the age and gender of a patient. That is, as the heart muscle decreases with age, the amplitude of the electrocardiogram tends to decrease. In the case of women, the electrode location of the electrocardiogram is lowered or the distance between the heart and the electrode is increased due to the breast, and thus the shape of the electrocardiogram is deformed.

In addition, in the case of chronic lung disease (chronic obstructive pulmonary disease), as the capacity of the lungs increases, the heart between the lungs is erected in a vertical direction, so that the electrical flow of the heart changes in a vertical direction in a three-dimensional space.

Accordingly, the training unit 130 inputs the lead electrocardiograms, stored separately according to the patient information, that is, the age, gender, health level, and potential vector of each patient, to the first training model and the second training models. Then, the first training model and the second training models are trained on the characteristics of the input electrocardiograms and extract the potential vector information of the input electrocardiograms. However, it may be also possible to receive electrocardiograms and be trained regardless of classification according to the patient information. Through this method, the training models that are not limited to one characteristic may be trained.

In addition, the training unit 130 constructs 12 second training models corresponding to 12-lead electrocardiograms. Furthermore, the training unit 130 trains each of the second training models according to a characteristic of the electrocardiograms.

In other words, the first training model is trained for the correlations between the input electrocardiograms and 12-lead electrocardiograms based on a deep learning algorithm, extracts at least one characteristic, i.e., age, gender, and potential vector, of the input electrocardiograms, and generates (12-n) lead virtual electrocardiograms from n lead virtual electrocardiograms through this. Meanwhile, the second training models generate n lead virtual electrocardiograms from the (12-n) lead virtual electrocardiograms. For example, assuming that a lead V1 electrocardiogram is generated, the training unit 130 trains the first training model on styles so that the first training model generates virtual lead I, II, III, aVL, aVR, aVF, V2, V3, V4, V5 and V1 electrocardiograms, and trains the second training models on styles so that the second training models generate virtual lead V1 electrocardiograms from virtual lead I, II, III, aVL, aVR, aVF, V2, V3, V4, V5, and V6 electrocardiograms. Then, the second training models generate virtual electrocardiograms by converting the input electrocardiograms into lead V1 style electrocardiograms.

In order to determine the type of lead of an input electrocardiogram, information about the type of lead of an electrocardiogram is also input to the first training model and the first training model is also trained on the information about the type of lead of the electrocardiogram. When the first training model is used after being trained through this, it may generate 12-lead electrocardiograms even when electrocardiograms whose types of lead are unknown are input.

In this case, the first training model and the second training models are based on a deep learning algorithm composed of an autoencoder or a generative adversarial network. The deep learning algorithm may be implemented using one selected from an autoencoder and a generative adversarial network, or may be implemented by mixing an autoencoder and a generative adversarial network.

When the training of the training models is completed through steps S210 and S230, the system 100 for generating electrocardiograms according to the embodiment of the present invention generates electrocardiograms using the trained training models.

First, the system 100 for generating electrocardiograms receives a reference electrocardiogram measured through electrodes attached to the body of a subject to be measured in step S240.

In this case, the reference electrocardiogram does not include information about the potential vector.

Thereafter, the electrocardiogram generation unit 140 generates a plurality of virtual electrocardiograms by inputting the reference electrocardiogram to the first training model and the second training models in step S250.

First, the electrocardiogram generation unit 140 extracts a characteristic of the reference electrocardiogram by inputting the reference electrocardiogram to the first training model.

In this case, the characteristic includes at least one of the age, gender, and potential vector of the subject to be measured.

Through this, a first virtual electrocardiogram is generated from the reference electrocardiogram.

Thereafter, the electrocardiogram generation unit 140 inputs the generated first virtual electrocardiogram to the second training models. Then, the second training models generate second virtual electrocardiograms based on the input first virtual electrocardiogram.

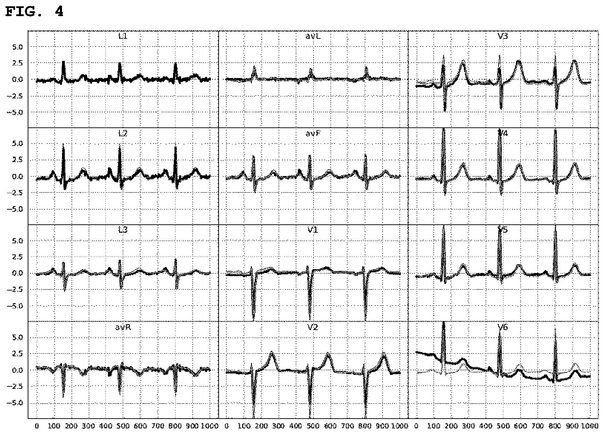

FIG. 4 is an exemplary diagram illustrating step S240 shown in FIG. 2.

As shown in FIG. 4, the first electrocardiogram represents a reference electrocardiogram. In this case, it is assumed that the first training model determines L1 to be a characteristic of the reference electrocardiogram. Then, the electrocardiogram generating unit 140 allows the first training model to generate the remaining 11 lead virtual electrocardiograms based on the L1 electrocardiogram. After the generated 11 lead virtual electrocardiograms have been input to the second training models, a virtual lead L1 electrocardiogram is generated. In each of the training models, a generator adapted to generate an electrocardiogram and a discriminator adapted to determine whether a generated electrocardiogram is accurately generated and increase the accuracy of the generated electrocardiogram through feedback are constructed together.

When step S250 is completed, the control unit 150 outputs the reference electrocardiogram and the 11 virtual electrocardiograms through the monitoring device in step S260.

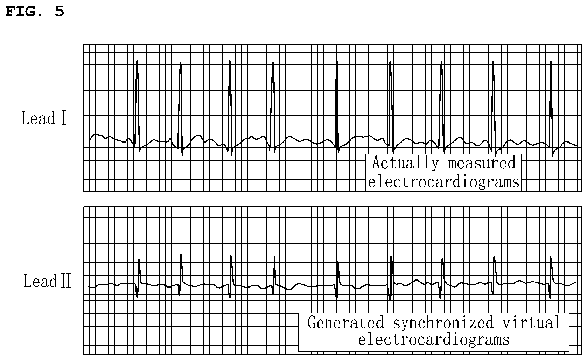

FIG. 5 is an exemplary view illustrating step S260 shown in FIG. 2.

The control unit 150 outputs the reference electrocardiogram and the 11 virtual electrocardiograms. Furthermore, as shown in FIG. 5, the control unit 150 synchronizes the output reference electrocardiogram and 11 virtual electrocardiograms with each other and determines whether there is an abnormality in the health of the subject to be measured. In this case, a virtual electrocardiogram having the same lead type as the reference electrocardiogram is also generated in the second training model, the above determination may be performed based on 12 virtual electrocardiograms.

As described above, there is output an electrocardiogram having a gradient, amplitude, and the like varying depending on the age, gender, and health of a subject to be measured. Accordingly, the control unit 150 outputs 12 electrocardiograms, synchronized via the reference electrocardiogram and the 11 virtual electrocardiograms, through the monitoring device.

As described above, according to the present invention, a plurality of synchronized electrocardiograms may be generated from two electrocardiograms, measured at different points, by using a deep learning algorithm, so that the disease of the heart can be accurately diagnosed as in a 12-lead electrocardiogram.

In addition, the system for generating electrocardiograms according to the present invention may perform measurement at home or in daily life because it uses two lead electrocardiograms, may enable real-time monitoring because it can perform measurement even in a moving state, and may enable medical staff to read synchronized lead electrocardiogram information tailored to a corresponding beat because synchronized electrocardiograms are generated utilizing electrocardiogram information measured at different times, thereby enabling more accurate diagnosis based on this.

While the present invention has been described with reference to the embodiments shown in the drawings, these are only examples. It will be understood by those skilled in the art that various modifications and other equivalent embodiments may be made therefrom. Therefore, the true technical protection range of the present invention should be determined by the technical spirit of the claims below.
- 100:: system for generating electrocardiograms
- 110:: data input unit
- 120:: data extraction unit
- 130:: training unit
- 140:: electrocardiogram generation unit
- 150:: control unit

### Industrial Applicability

The present invention may generate a plurality of synchronized electrocardiograms from two electrocardiograms, measured at different points, by using a deep learning algorithm, so that the disease of the heart can be accurately diagnosed as in a 12-lead electrocardiogram. The present invention is industrially applicable to systems for generating electrocardiograms based on various deep learning algorithms.

## Claims

1. A system for generating electrocardiograms based on a deep learning algorithm, the system comprising:
a data input unit configured to receive 12-lead electrocardiograms measured by a plurality of patients;
a data extraction unit configured to extract training data from the input 12-lead electrocardiograms;
a training unit configured to train a plurality of training models on a characteristic of the electrocardiograms by inputting the extracted training data to the plurality of training models;
an electrocardiogram generation unit configured to receive at least one reference electrocardiogram from a subject to be measured, and to generate one or more virtual electrocardiograms by inputting the input reference electrocardiogram into the plurality of trained training models; and
a control unit configured to synchronize the reference electrocardiogram and the generated virtual electrocardiograms with each other, and to output waveforms for the synchronized reference electrocardiogram and virtual electrocardiograms.

2. The system of claim 1, wherein the patient information includes at least one of gender, age, whether there is a heart disease, and a potential vector of each of the measured electrocardiograms.

3. The system of claim 2, wherein the training unit trains a first training model to determine a potential vector for each of the input electrocardiograms by inputting 6-lead limb electrocardiograms and 6-lead chest electrocardiograms to the first training model.

4. The system of claim 3, wherein the training unit:
constructs a first training model and second training models for 6-lead limb electrocardiograms and 6-lead chest electrocardiograms; and
trains the constructed first and second training models to, when inputting one or more reference lead electrocardiograms to the first and second training models, convert the input reference lead electrocardiograms into styles each having a corresponding potential vector and output them as virtual electrocardiograms.

5. The system of claim 3 or 4, wherein the first and second training models are each constructed by mixing a generative adversarial network and an autoencoder method or using each of them.

6. The system of claim 5, wherein the electrocardiogram generation unit:
extracts a potential vector for the reference electrocardiogram and generates a first virtual electrocardiogram by inputting the input reference electrocardiogram to the first training model; and
generates second virtual electrocardiograms by inputting the first virtual electrocardiogram to the second training models.

7. The system of claim 6, wherein the control unit:
synchronizes the reference electrocardiogram and the plurality of virtual electrocardiograms by matching them;
outputs the plurality of synchronized electrocardiograms through a monitor; and
determines whether there is an abnormality in health of the subject to be measured by using at least one of an amplitude, gradient, and electrode position of each of the output waveforms for the reference electrocardiogram and virtual electrocardiograms.

8. A method for generating electrocardiograms using a system for generating electrocardiograms, the method comprising:
receiving 12-lead electrocardiograms measured by a plurality of patients and patient information corresponding to the 12-lead electrocardiograms;
classifying and storing the input 12-lead electrocardiograms according to the patient information, and extracting training data from the stored 12-lead electrocardiograms;
training a plurality of training models on a characteristic of the electrocardiograms by inputting the extracted training data to the plurality of training models;
receiving at least one reference electrocardiogram from the subject to be measured, and generating one or more virtual electrocardiograms by inputting the input reference electrocardiogram into the plurality of trained training models; and
synchronizing the reference electrocardiogram and the generated virtual electrocardiograms with each other, and outputting waveforms for the synchronized reference electrocardiogram and virtual electrocardiograms and information about whether there has occurred an abnormality in health of the subject to be measured.

9. The method of claim 8, wherein the patient information includes at least one of gender, age, whether there is a heart disease, and a potential vector of each of the measured electrocardiograms.

10. The method of claim 8, wherein training the plurality of training models on the characteristic of the electrocardiograms includes training a first training model to determine a potential vector for each of the input electrocardiograms and generate a first virtual electrocardiogram by inputting 6-lead limb electrocardiograms and 6-lead chest electrocardiograms to the first training model.

11. The method of claim 10, wherein training the plurality of training models on the characteristic of the electrocardiograms includes:
constructing second training models trained for styles based on the first virtual electrocardiogram; and
training the constructed second training models to, when inputting the first virtual electrocardiogram to the second training models, convert the input lead electrocardiograms into styles each having a corresponding potential vector and output them as second virtual electrocardiograms.

12. The method of claim 10 or 11, wherein the first and second training models are each constructed by mixing a generative adversarial network and an autoencoder method or using each of them.

13. The method of claim 12, wherein generating the virtual electrocardiograms includes:
extracting a potential vector for the reference electrocardiogram and generating a first virtual electrocardiogram by inputting the input reference electrocardiogram to the first training model; and
generating second virtual electrocardiograms by inputting the first virtual electrocardiogram to the second training models that have been trained on the extracted potential vector.

14. The method of claim 13, wherein outputting the information about whether there has occurred the abnormality in the health of the subject to be measured includes:
synchronizing the reference electrocardiogram and the plurality of virtual electrocardiograms by matching them;
outputting the plurality of synchronized electrocardiograms through a monitor; and
determining whether there is an abnormality in health of the subject to be measured by using at least one of an amplitude, gradient, and electrode position of each of the output waveforms for the reference electrocardiogram and virtual electrocardiograms.
